# EUROPEAN PATENT SPECIFICATION

(11) **EP 2 872 842 B1**
(45) Date of publication and mention of the grant of the patent: **03.04.2019**
(21) Application number: 13816501.4
(22) Date of filing: 12.06.2013
(51) Int. Cl.: F25J 3/02

(54) **METHODS FOR SEPARATING HYDROCARBON GASES**
VERFAHREN ZUR TRENNUNG VON KOHLENWASSERSTOFFGASEN
PROCÉDÉS POUR LA SÉPARATION DE GAZ HYDROCARBONÉS

(30) Priority: 12.07.2012 US 201213547153
(43) Date of publication of application: 20.05.2015
(73) Proprietor: Linde Engineering North America Inc., Blue Bell, PA 19422 (US)
(72) Inventor: MALIK, Zaheer, I., Tulsa, OK 74137 (US); KEY, Ronald, D., Broken Arrow, OK 74011-1536 (US)
(74) Representative: Gellner, Bernd
(86) International application number: PCT/US2013/045346
(87) International publication number: WO 2014/011344

(56) References cited:
- US-A- 4 895 584
- US-A1- 2002 042 551
- US-A1- 2004 148 964
- US-A1- 2004 206 112
- US-A1- 2007 157 663
- US-B1- 6 311 516

## Description

### BACKGROUND OF THE INVENTION

The present invention relates to a method and apparatus for the improved recovery of C2 and/or C3 and heavier components from hydrocarbon gases. US 2004/0148964, which can be considered as the closest prior art, describes a process for separating a hydrocarbon gas using a heavy ends fractionation column and a lights end fractionation column.

In conventional processes for extracting propane and C2 and/or C3 bearing gases are treated by a combination of expansion of heavier components from hydrocarbon gases (or compression followed by expansion) heat exchange and refrigeration to obtain a partially condensed stream which is collected in a feed separator having a pressure typically in the order of 50 to 1100 psia and a temperature in the order of -50° to -200° F. These conditions of course can vary substantially, depending on the pressure and temperature conditions necessary to achieve partial condensation for a particular gas, and the pressure and temperature at which the feed is available to the process. The liquid resulting from partial condensation is supplied to a fractionation column called a "heavy ends fractionation column" as a mid-column feed while the vapor from the feed separator is used to generate reflux by partially condensing the overhead vapors from the heavy ends fractionation column through appropriate heat exchange means. In a typical system the heavy ends fractionation column will operate at a pressure less than that of the feed separator (possibly allowing for a small pressure drop as the partially condensed liquid passes from the separator to the heavy ends fractionation column) and the heavy ends fractionation column overhead vapors leave at a temperature in the order of -120° to -160°F for C2 and heavier recovery and - 20° to - 70° F for C3 and heavier recovery. The heat exchange of these overhead vapors against the residue vapors from the light ends fractionation column provides partial condensate which is used as a reflux to the light ends fractionation column.

Pre-cooling of the gas before it is expanded to the light ends fractionation column pressure will commonly result in formation of a high-pressure condensate. To avoid damage to the expander, the high pressure condensate, if it forms, is usually separated in the feed separator, separately expanded through a Joule-Thomson valve and used as a further feed to the mid-portion of the heavy ends fractionation column.

Refrigeration in such a process is sometimes entirely generated by work expansion of the vapors remaining after partial condensation of the high pressure gas to the light ends fractionation column operating pressure. Other processes may include external refrigeration of the high pressure gases to provide some of the required cooling.

When processing natural gas, feed is typically available at line pressure, of 900 to 1100 psia. In such case expansion to a pressure in the order of 150 to 500 psia is common. In an alternate process, facilities may be designed to extract propane or propylene from refinery gases. Refinery gases commonly are available a pressure of 50 psia to 250 psia. In this case, at the convenience of the process designer, the light ends fractionation column may be designed to operate at a pressure below the pressure of the refinery gas which is available, i.e., perhaps 50 to 100 psia, so that work expansion can be used to supply refrigeration to the process. This will result in lower light ends fractionation column temperatures and will increase potential heat leakage and other engineering problems associated with cryogenic temperatures, It is also possible in this case to compress the refinery gas to a higher pressure so that it may be thereafter expanded in a work-expansion machine to afford refrigeration to the overall process.

A typical flow plan of a process for separating C3 and heavier hydrocarbons from a gas stream is illustrated in U.S. Pat. No. 4,251,249 to Jerry G. Gulsby.

### SUMMARY OF THE INVENTION

A process according to the invention is defined in claim 1.

The contacting step (2) is carried out in a feed separator/absorber which includes fractionation means for vapor/liquid counter-current contact and
(i) wherein said partly condensed second residue is introduced into said separator/absorber above or at an intermediate point in said fractionation means, whereby the liquid portion of it passes downwardly through said fractionation means; and
(ii) wherein said partly condensed portion of the first residue is introduced into said separator/absorber above or at an intermediate point in said fractionation means, whereby the liquid portion of it passes downwardly through said fractionation means; and wherein said portion of the cooled C2 or C3 containing liquid from the separator is introduced into said separator/absorber above or at an intermediate point in said fractionation means, whereby the liquid portion of it passes downwardly through said fractionation means; and
(iii) said at least part of one of said first residue vapors is supplied to said separator/absorber below said fractionation means, whereby the first residue vapor rises through said fractionation means in counter-current contact with the liquid portion of the partly condensed second residue.

The fractionation means in said separator/absorber provide the equivalent of at least one theoretical distillation stage arranged to contact at least part of one of said first residue vapors with the liquid portion of the partly condensed second residue.

The fractionation means in said separator/absorber provide the equivalent of at least three theoretical distillation stages arranged to contact at least part of one of said first residue vapors with the liquid portion of the partly condensed second residue.

At least part of one of said first residue vapors are co-mingled with the liquid portion of the partially condensed second residue, liquid portion of the partially condensed portion of the first residue and portion of the cooled C2 or C3 containing liquid from the separator.

At least part of one of said first residue vapors are comingled with both the liquid portions and vapor portions of said partially condensed second residue, partially condensed portion of the first residue vapor and portion of the cooled C2 or C3 containing liquid from the separator,

Further, the invention includes an apparatus according to claim 6.

The contacting and separating means includes fractionation means for countercurrent vapor/liquid contact and wherein said means is connected to receive the portion of one of first residue vapors to be treated therein below said fractionation means and to receive the portion of said liquids from the partially condensed second residue, portion of the partially condensed first residue and portion of the cooled C2 or C3 containing liquid from the separator to be treated therein above or at an intermediate point in said fractionation means said fractionation means thereby being adapted so that the first residue vapors rise there-through in countercurrent contact with partially condensed second residue and portion of the partially condensed first residue and being further adapted so that the portion of the C2 or C3 containing liquid from the separator is cooled by the liquids exiting the fractionation means.

The fractionation means includes vapor/liquid contacting means which are the equivalent of at least one theoretical distillation stage.

The contacting and separating means (2) comprise means for comingling at least part of one of said first residue vapors with the liquid portion of the partially condensed second residue, liquid portion of the partially condensed portion of the first residue and portion of the cooled C2 or C3 containing liquid from the separator.

The contacting and separating means (2) comprise means for comingling at least part of one of said first residue vapors with both the liquid and vapor portion of said partially condensed second residue, said partially condensed portion of the first residue and portion of the cooled C2 or C3 containing liquid from the separator.

### BRIEF DESCRIPTION OF THE DRAWINGS

Figures 1A and 1B are schematics of a hydrocarbons separation process not according to the invention.
Figures 2A and 2B are schematics of an example of a hydrocarbons separation process not according to the invention.
Figures 3A and 3B are schematics of a preferred embodiment of a hydrocarbons separation process according to the invention.

### DETAILED DESCRIPTION OF THE INVENTION

The present invention provides a process for recovering C2 and/or C3 and heavier components from hydrocarbon-bearing gases. In the process of the present invention the overhead vapor from the heavy ends fractionation column and a portion of the first residue vapor from the separator are partly condensed and at least a portion of the C2 or C3 containing liquid from the separator into a heat exchange relationship with the liquid product from the contacting device and then at least the respective liquid condensates and cooled liquid are combined with at least the vapor from the partially condensed feed gases described above in the heavy ends fractionation column feed separator which, in the present invention, also acts as an absorber. The feed separator/absorber is designed to afford one or more contacting stages. Usually such stages are assumed for design purposes to be equilibrium stages, but in practice this need not be so. Vapor from the feed separator/absorber passes in heat exchange relation to the overhead from the heavy ends fractionation column, thereby providing partial condensation of the heavy ends fractionation column overhead vapor and portion of the first residue vapor, and liquid from the feed separator/absorber is supplied to the heavy ends fractionation column as an upper or top liquid feed to the column.

If the separator/absorber contains an absorption section, such as packing, or one or more fractionation trays, these stages will be assumed to correspond to a suitable number of theoretical separation stages. Our calculations have shown benefits with as few as one theoretical stage, and greater benefits as the number of theoretical stages is increased. We believe that benefits can be realized even with the equivalent of a fractional theoretical stage. The partially condensed heavy ends fractionation column overhead , partially condensed portion of the first residue vapor, and at least a portion of the cooled C2 or C3 containing liquid from the separator are supplied above or at an intermediate point of this section, and the liquid portions of these streams passes downward through the absorption section. The partially condensed feed stream is usually supplied below the absorption section, so that the vapor portion of it passes upwardly through it in countercurrent contact with the liquids from the partially condensed heavy ends fractionation column overhead. The rising vapor joins the vapors which separate from partially condensed heavy ends fractionation column overhead above the absorption section, to form a combined residue stream.

While described above with respect to a preferred embodiment in which overhead, a portion of the first residue vapors are condensed and, at least a portion of the cooled C2 or C3 containing liquid from the separator are used to absorb valuable ethane, propane, etc. from the expander outlet vapors, we point out that the present invention is not limited to this exact embodiment. Advantages can be realized, for instance, by treating only a part of the expander outlet vapor in this manner, or using only part of the overhead condensate or none of the separator liquid as an absorbent in cases where other design considerations indicate that portions of the expander outlet or overhead condensate should bypass the feed separator-/absorber. We also point out that the feed separator/absorber can be constructed as either a separate vessel, or as a section of the heavy ends fractionation column.

In the practice of this invention there will necessarily be a pressure difference between the separator/absorber and the heavy ends fractionation column which must be taken into account. If the overhead vapors pass through the condenser and into the separator without any boost in pressure, the feed separator/absorber will assume an operating pressure slightly below the operating pressure of the heavy ends fractionation column. In this case the liquid feed withdrawn from the separator/absorber can be pumped to its feed position in the heavy ends fractionation column. An alternative is to provide a booster blower in the vapor line to raise the operating pressure in the overhead condenser and separator/absorber sufficiently so that the liquid feed can be supplied to the heavy ends fractionation column without pumping. Still another alternate is to mount the feed separator/absorber at a sufficient elevation relative to the feed position of the liquid withdrawn therefrom that the hydrostatic head of the liquid will overcome the pressure difference.

In still another alternative, all or a part of the partially condensed heavy ends fractionation column overhead and all or part of the partially condensed feed can be combined, such as in the pipe line joining the expander output to the feed separator/absorber and if thoroughly intermingled, the liquids and vapors will mix together and separate in accordance with a relative volatility of the various components of the total combined streams. In this embodiment the vapor-liquid mixture from the overhead condenser can be used without separation, or the liquid powder thereof may be separated. Such co-mingling is considered for purposed of this invention as a contacting stage.

In still another variation of the foregoing, the partially condensed overhead vapors can be separated, and the all or a part of the separated liquid supplied to the separator/absorber or mixed with the vapors fed thereto.

The present invention provides improved recovery of propane or propylene per amount of horsepower input required to operate the process. An improvement in operating horsepower required for operating a heavy ends fractionation column process may appear either in the form of reduced power requirements for external refrigeration, reduced power requirements for compression or recompression, or both. Alternatively, if desired, increased C3 recovery can be obtained for a fixed power input.

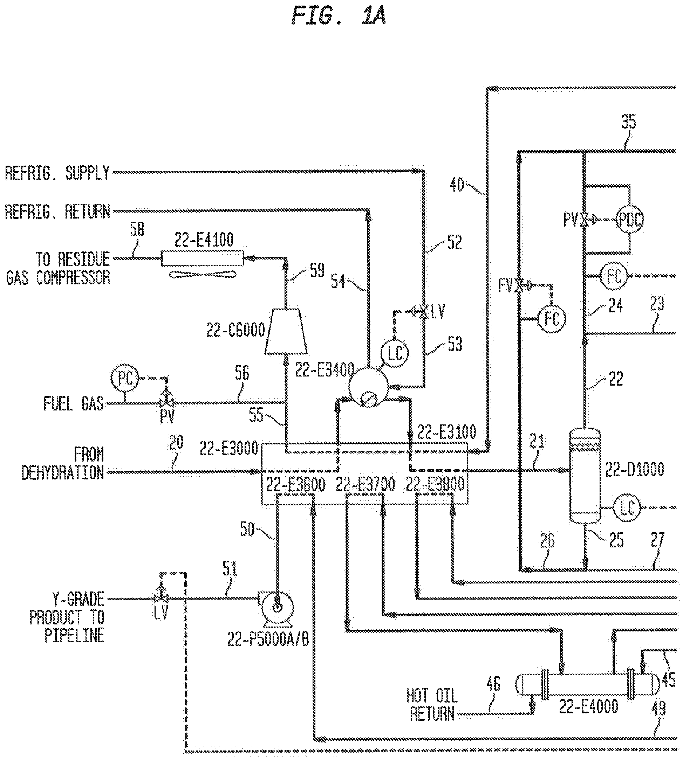

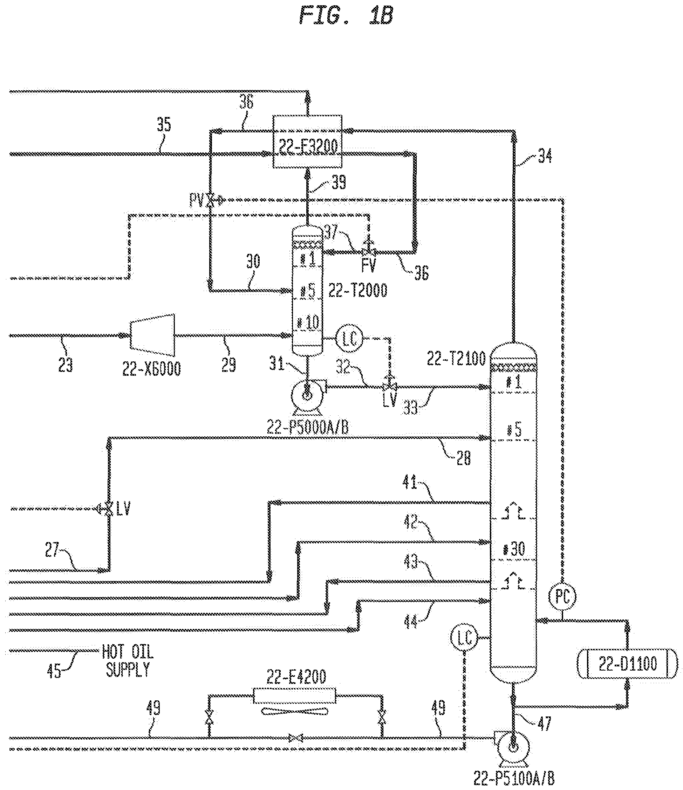

Turning to the figures, Figures 1A and 1B are schematics of a hydrocarbon separation process. A hydrocarbon bearing gas natural gas is fed through line 20 to a warm gas/gas exchanger 22-E3000 and then to a chiller 22-E3400. Refrigeration is supplied through line 52 and 53 with some refrigerant removed through a valve assembly before entering the chiller. The chiller has a line 54 which will withdraw refrigeration for recompression and liquefaction. The cooled gas stream is fed through line 21 through a cold gas/gas exchanger 22-3100 to a cold separation column 22-D1000.

The hydrocarbon gas stream will be separated into two streams with the tops leaving through line 22 and the bottoms through line 25 to line 16. The bottoms will pass through a valve in line 26 for flow control and will rejoin line 26 to line 35 where they will enter subcooler 22-E3200. These cooled hydrocarbon gases leave the subcooler through line 36 and enter light ends fractionation column 22-T2000. The hydrocarbon gas stream that is not diverted will continue through line 37 to the light ends fractionation column 22-T2000 at the top of the column.

The tops from the cold separation column 22-D1000 will leave through line 22 and reach a junction with line 24. Line 24 will also contain a valve assembly PV which is used to control the flow of the stream in Line 24. The remainder of the tops from the cold separation column flow through line 23 through an expander/compressor 22-X6000. This expanded hydrocarbon gas stream will be fed through line 29 into the light ends fractionation column 22-T2000.

The tops from the light ends fractionation column 22-T2000 will leave through line 39 and pass through line 40 where they will pass through cold gas/gas exchanger 22-E3100 and warm gas/gas exchanger before passing through line 55 to an expander/compressor 22-C6000 where the compressed gas stream will enter and expander/compressor discharge cooler 22-E4100 through line 59. The discharged gas stream will exit through line 58 and for sales or further processing as required.

Line 56 contacts line 55 and some of the hydrocarbon gas will be drawn off before entering the expander/compressor 22-C6000 and recovered for use as fuel gas. A valve assembly is present in line 56 for controlling the quantity of the material to be used as fuel gas.

The bottoms from the light ends fractionation column 22-T2000 will exit through line 31. These bottoms comprise an intermediate liquid stream that required further fractionation. Line 31 is in fluid communication with a transfer pump 22-P5000A/B which directs the bottoms from the light ends fractionating column to line 33 and into the top of a heavy ends fractionation column 22-T2100.

Part of the bottoms from the cold separator column 22-D1000 are diverted through line 27 where they will pass through a level control valve that flows through line 28 into the heavy ends fractionating column.

A stream comprising a cooler, intermediate product liquid is withdrawn from the heavy ends fractionation column 22-T2100 through line 41 which is fed to a side heater 22-E3800 which will heat the stream and return it through line 42 to a point lower in the heavy ends fractionation column from which it was withdrawn. Another side steam is withdrawn from the heavy ends fractionation column 22-T2100 through line 43 which is fed to a heavy ends fractionation column reboiler 22-E3700 which will heat the side stream. This stream is fed to a trim reboiler 22-E4000 where it will be further heated before being returned through line 44 to a point lower in the heavy ends fractionation column from which it was withdrawn. Line 45 will supply hot oil from a hot oil supply (not shown) to the trim reboiler 22-E4000 while line 46 will return hot oil from the trim reboiler.

A line at the bottom of the heavy ends fractionating column will remove some of the hydrocarbon comprising mainly of C2s and less volatile hydrocarbons or C3s and less volatile hydrocarbon and direct it to a valve in line 51. Line 51 receives bottoms from the heavy ends fractionating column 22-T2100. Line 47 feeds the bottoms from the heavy ends fractionating column and feeds them to a heavy ends fractionating column bottoms pump 22-P5100A/B which feeds the bottoms through line 49 to a product exchanger 22-E3600 which feeds the bottoms through line 50 to the product pump 22-P5200A/B. This pump directs the bottoms through line 51 where they can be directly fed to a pipeline.

A valve in line 49 will allow bypass of the product exchanger 22-E3600 and divert the flow to an air or water cooled heat exchanger when the plant is operated in the C3 and heavier recovery mode. After cooling, these bottoms can be fed back into line 49 for feeding to the product exchanger 22-E3600.

The tops from the heavy ends fractionation column 22-T2100 will exit through line 34 and pass through a subcooler 22-E3200. Line 38 exits the subcooler 22-E3200 and connects to a valve PV. The tops from the heavy ends fractionation column will be fed through line 30 into the light ends fractionation column 22-T2000 where they will be further fractionated for reentry back into the heavy ends fractionation column as a reflux stream.

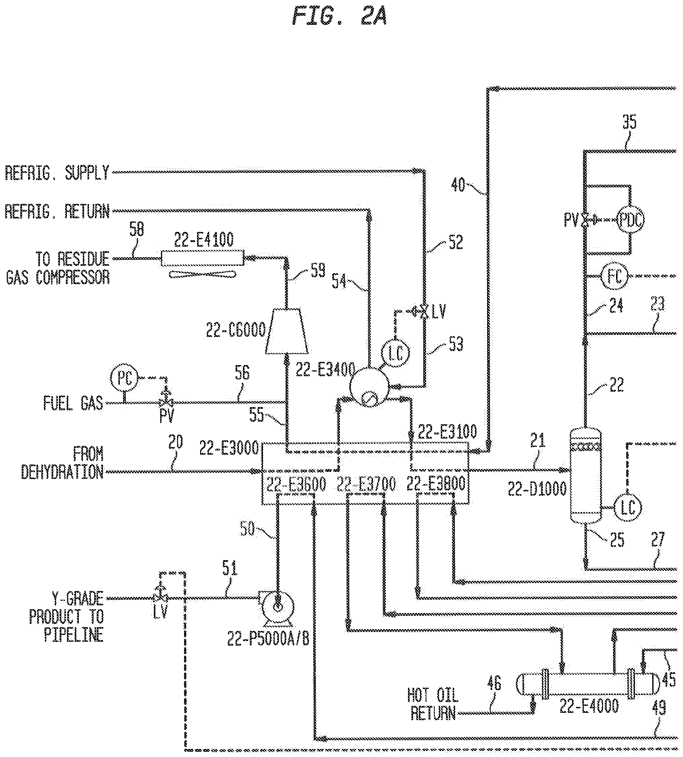

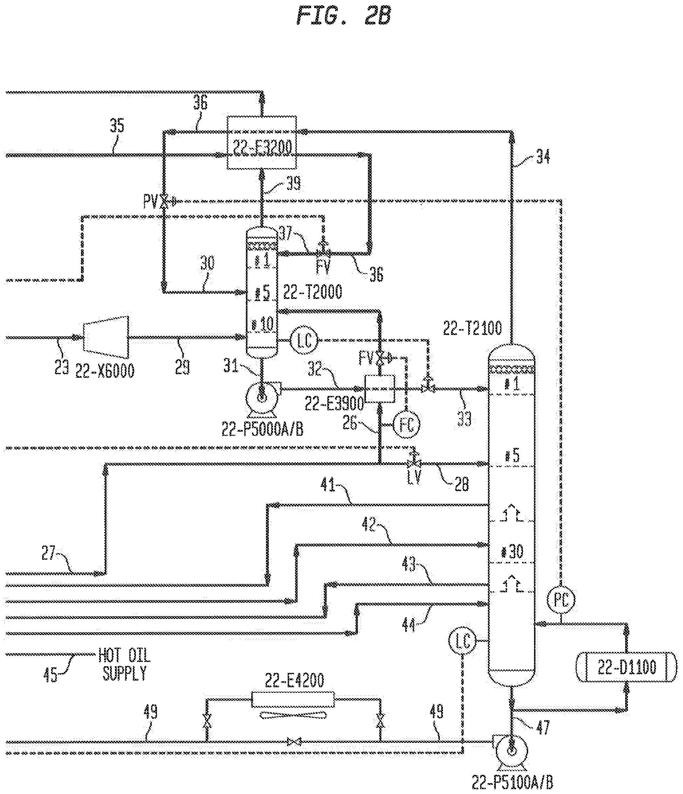

Figures 2A and 2B represent an alternative example not forming part of the present invention. In this alternative description all the designations as employed in describing Figures 1A and 1B are re-employed and mean the same for the description of the unit operations taking place. In Figures 2A and 2B, a liquid/liquid exchanger is present between the heavy ends fractionation column and the light ends fractionation column.

The bottoms from the cold separator column 22-D1000 will be fed through line 25 to a junction connecting to a valve LV and line 28 for entry into the heavy ends fractionation column. The feed through line 26 will connect with a liquid/liquid exchanger 22-E3900 and pass through into the light ends fractionation column 22-T2000.

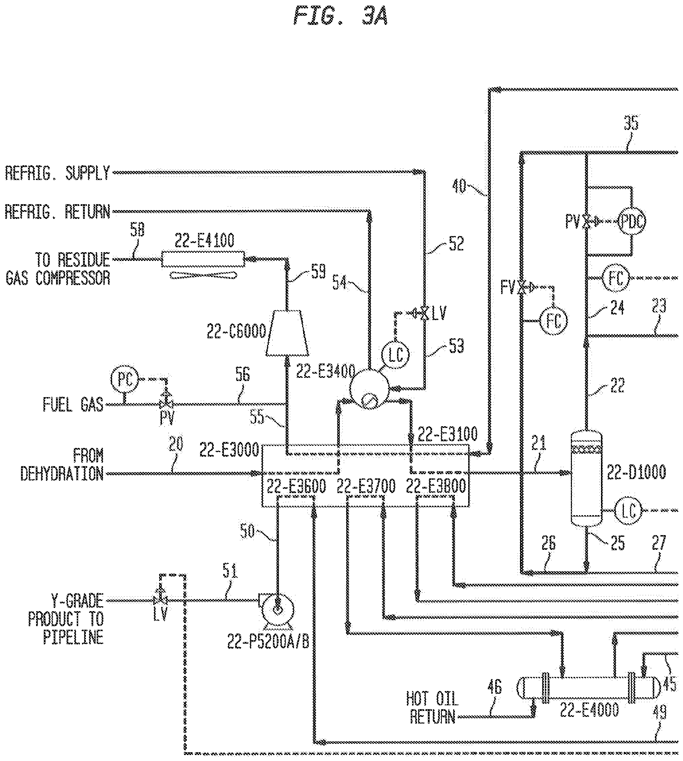

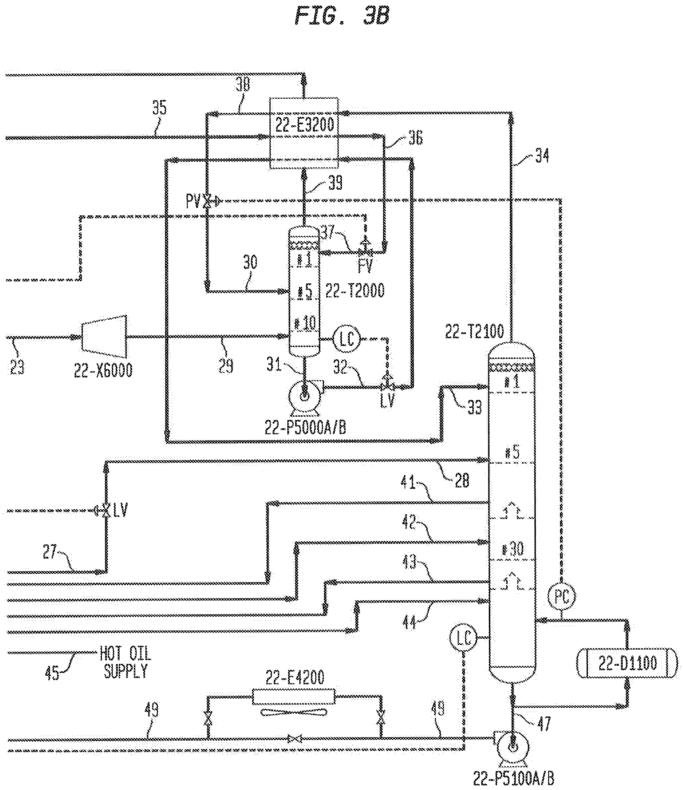

Figures 3A and 3B represent another alternative embodiment of the present invention, In this alternative description all the designations as employed in describing Figures 1A and 1B are re-employed and mean the same for the description of the unit operations taking place. In Figures 3A and 3B, the bottoms from the light ends fractionation column 22-T2000 are fed through line 31 to the light ends fractionation column bottoms pump 22-P5000A/B which feeds the bottoms through line 32 and valve LVI to subcooler 22-E3200. Valve LVI may be opened and closed to divert some of the bottoms back to the bottom of the light ends fraction column.

The bottoms fed to the subcooler 22-E3200 are now lower in temperature and are fed through line 33 into heavy ends fractionation column 22-T2100 where they will be further fractionated.

## Claims

1. A process for separating a hydrocarbon gas containing at least methane, ethane and C3 components into a fraction containing a predominant portion of the ethane and lighter components and a fraction containing a predominant portion of the C3 and heavier components or a predominant portion of the methane and lighter components and a fraction containing a predominant portion of the C2 and heavier components, in which process
(a) the feed gas (20) is treated in one or more heat exchangers (22-E3600, 22-E3700, 22-E3800), and expansion steps to provide at least one partly condensed hydrocarbon gas, which is sent to a separator (22-D1000) providing thereby at least one first residue vapor (22) and at least one C2 or C3-containing liquid which liquid (25) also contains lighter hydrocarbons; and
(b) at least one of the C2 or C3-containing liquids is directed (25 to 27 to 28) into a heavy ends fractionation column (22-T2100) wherein said liquid is separated into a second residue (34) containing lighter hydrocarbons and a C2 or C3-containing product; comprising:
(1) cooling (22-E3200) said second residue (34) to partially condense said second residue;
(2) intimately contacting at least part of one of said first residue vapors (22) with at least part of a liquid portion of the partially condensed second residue (30) in at least one contacting stage (#1 #5 #10) of a light ends fractionation column (22-T2000) and thereafter separating vapors (39) and liquids (31) from said contacting stage;
(3) directing the vapors (39) from said contacting stage (#1 #5 #10) of the light ends fractionation column (22-T2000) into heat exchange relation (22-E3200) with said second residue (34) from the heavy ends fractionation column (22-T2100), thereby to supply the cooling of step (1), and thereafter discharging (39 to 40) said vapors from said contacting stage of the light ends fractionation column (22-T2000);
(4) withdrawing a portion of the first residue vapor (22);
(5) cooling (22-E3200) said portion of the first residue vapor (22, 35) to partially condense said portion of said first residue vapor;
(6) intimately contacting (#1 #5 #10) the at least part of one of said first residue vapors from step (2) with at least part of a liquid portion of the partially condensed portion of the first residue from step (5) in the at least one contacting stage of the light ends fractionation column (22-T2000) and thereafter separating the vapors (39) and liquids (31) from said contacting stage;
(7) withdrawing a portion of the C2 or C3-containing liquid (25) from the separator (22-D1000);
(8) directing (25, 26, 35) said portion of the C2 or C3-containing liquid from the separator (22-D1000) into a heat exchange relationship (22-E3200) with the liquids (31, 32) from the contacting stage of the light ends fractionation column (22-T2000);
(9) cooling (22-E3200) said portion of the C2 or C3-containing liquid (35) from the separator (22-D1000);
(10) intimately contacting the at least part of one of said first residue vapors (22, 23, 29) of step (2) with at least part of the C2 or C3-containing liquid (36) from the separator (22-D1000) in the at least one contacting stage (#1 #5 #10) of the light ends fractionation column (22-T2000) by feeding (36, 37) the at least part of one of said first residue vapors with at least part of the C2 or C3-containing liquid to a the light ends fractionation column (22-T2000) at a point higher (37) than (34, 38, 30) the feed of the liquid portion of the partially condensed second residue (38, 30) from the heavy ends fractionation column (22-T2100) and thereafter separating the vapors and liquids from said contacting stage;
(11) supplying the liquids (32, 33) from said contacting stage (#1 #5 #10) of the light ends fractionation column (22-T2000) of step (8) to the heavy ends fractionation column (22-T2100) as a liquid feed thereto;
(12) directing the vapors (39) from said contacting stage (#1 #5 #10) of the light ends fractionation column (22-T2000) into heat exchange relation (22-E3200) with said portion of the first residue vapors (22, 35) from the separator (22-D1000), thereby to supply the cooling of step (5), and thereafter discharging (39, 40) said vapors from said contacting stage (#1 #5 #10) of the light ends fractionation column (22-T2000); and
(13) directing the liquids (31, 32) from said contacting stage (#1 #5 #10) of the light ends fractionation column (22-T2000) into heat exchange relation with said portion of the C2 or C3-containing liquid (25, 26, 35) from the separator (22-D1000), thereby to supply the cooling of step (9), and thereafter discharging said liquids (33) from said contacting stage of the light ends fractionation column (22-T2000) to the heavy ends fractionation column (22-T2100).

2. The process according to claim 1 wherein said contacting step (2) is carried out in a light ends fractionation column (22-T2000) which includes fractionation means for vapor/liquid counter-current contact and
(i) wherein said partly condensed second residue (34) is introduced into said light ends fractionation column (22-T2000) above or at an intermediate point (30) in said fractionation means of the light ends fractionation column (22-T2000), whereby the liquid portion of it passes downwardly through said fractionation means; and
(ii) wherein said partly condensed portion of the first residue (22, 35) is introduced into said light ends fractionation column (22-T2000) above or at an intermediate point (37) in said fractionation means, whereby the liquid portion of it passes downwardly through said fractionation means; and wherein said portion of the cooled C2 or C3-containing liquid (25) from the separator (22-D1000) is introduced into said light ends fractionation column (22-T2000) above or at an intermediate point (37) in said fractionation means, whereby the liquid portion of it passes downwardly through said fractionation means; and
(iii) said at least part of one of said first residue vapors (22, 35) is supplied to said light ends fractionation column (22-T2000) below said fractionation means, whereby the first residue vapor rises through said fractionation means in counter-current contact with the liquid portion of the partly condensed second residue.

3. The process according to claim 2 wherein the fractionation means in said light -ends fractionation column (22-T2000) provide the equivalent of at least one theoretical distillation stage arranged to contact at least part of one of said first residue vapors (22, 23, 29) with the liquid portion of the partly condensed second residue (34, 38, 30).

4. The process according to claim 1 wherein at least part of one of said first residue vapors (22, 23, 29) are co-mingled with the liquid portion of the partially condensed second residue (34, 38, 30), liquid portion of the partially condensed portion of the first residue and portion of the cooled C2 or C3-containing liquid from the separator (22-D1000).

5. The process according to claim 1 wherein at least part of one of said first residue vapors are comingled in the light ends fractionation column (22-T2000) with both the liquid portions and vapor portions of said partially condensed second residue, partially condensed portion of the first residue vapor and portion of the cooled C2 or C3-containing liquid from the separator (22-D1000).

6. A system for separating a hydrocarbon gas containing at least methane, ethane and C3 components into a fraction containing a predominant portion of methane or ethane and lighter components and a fraction containing a predominant portion of the C2 or C3 and heavier components according to the process of claim 1 in which system
(a) one or more first heat exchange means (22-E3600, 22-E3700, 22-E3800) are in communication with one or more expansion means (22-X6000) to provide at least one partly condensed hydrocarbon gas which is sent to a separator (22-D1000), providing thereby at least one first residue vapor (22) and at least one C2 or C3-containing liquid (25) which liquid also contains lighter hydrocarbons; and
(b) a heavy ends fractionation column (22-T2100) connected to receive at least one of said C2 or C3-containing liquids (33) which is adapted to separate the C2 or C3-containing liquids into a second residue containing lighter hydrocarbons and a C2 or C3-containing product (34); further comprising:
(1) second heat exchange means (22-E3200) connected to said heavy ends fractionation column (22-T2100) to receive said second residue and to partially condense said second residue (34);
(2) said second heat exchange means (22-E3200) connected to said separator (22-D1000) to receive said a portion of the first residue vapor (22) and to partially condense said portion of said first residue;
(3) light ends fractionation column means (22-T2000) connected to receive at least part of one of the first residue vapors (36) and at least part of liquid portions from the partially condensed second residue (34) and partially condensed first residue vapor (36) and to comingle said at least part of one of the first residue vapors and the liquid portions of the partially condensed second residue and partially condensed first residue vapor in at least one contacting stage, said light ends fractionation column means (22-T2000) including separation means (#1, #5, #10) for separating the vapor and liquid from said contacting stage after contact in said stage;
(4) said light ends fractionation column means (22-T2000) being further connected to supply the liquid from said contacting stage to the heavy ends fractionation column (22-T2100) as a liquid feed thereto;
(5) said light ends fractionation column means (22-T2000) also being connected to direct the vapors from said contacting stage (39) into heat exchange relation with said second residue (36) and portion of the first residue (34) from the heavy ends fractionation column (22-T2100) and the separator (22-D1000) respectively in said second heat exchange means (22-E3200); and
(6) third heat exchange means (22-D1100) connected to said heavy ends fractionation column (22-T2100) to receive said liquids from said contacting stage (33) and to cool the portion of the C2 or C3-containing liquid from the separator (22-D1000).

7. The apparatus according to claim 6 wherein said light ends fractionation column means (22-T2000) includes fractionation means (#1, #5, #10) for countercurrent vapor/liquid contact and wherein said means is connected to receive the portion of one of first residue vapors to be treated therein below said fractionation means and to receive the portion of said liquids from the partially condensed second residue, portion of the partially condensed first residue and portion of the cooled C2 or C3-containing liquid from the separator (22-D1000) to be treated therein above or at an intermediate point in said fractionation means said fractionation means thereby being adapted so that the first residue vapors rise there-through in countercurrent contact with partially condensed second residue and portion of the partially condensed first residue and being further adapted so that the portion of the C2 or C3-containing liquid from the separator is cooled by the liquids exiting the fractionation means.

8. The apparatus according to claim 7 wherein said fractionation means (#1, #5, #10) includes vapor/liquid contacting means which are the equivalent of at least one theoretical distillation stage.

9. The apparatus according to claim 6 wherein said light ends fractionation column means (22-T2000) comprise means for comingling at least part of one of said first residue vapors with the liquid portion of the partially condensed second residue, liquid portion of the partially condensed portion of the first residue and portion of the cooled C2 or C3-containing liquid from the separator (22-D1000).

10. The apparatus according to claim 6 wherein said light ends fractionation column means (22-T2000) comprise means for comingling at least part of one of said first residue vapors with both the liquid and vapor portion of said partially condensed second residue, said partially condensed portion of the first residue and portion of the cooled C2 or C3-containing liquid from the separator (22-D1000).

## Patentansprüche

1. Verfahren zur Trennung eines Kohlenwasserstoffgases enthaltend mindestens Methan, Ethan und C3-Komponenten in eine Fraktion, die einen vorwiegenden Anteil des Ethans und leichterer Komponenten enthält, und eine Fraktion, die einen vorwiegenden Anteil des C3 und schwererer Komponenten oder einen vorwiegenden Anteil des Methans und leichterer Komponenten enthält, und eine Fraktion die einen vorwiegenden Anteil des C2 und schwererer Komponenten enthält, bei welchem Verfahren
(a) das Zufuhrgas (20) in einem oder mehr Wärmetauschern (22-E3600, 22-E3700, 22-E3800) und durch Expansionsschritte behandelt wird, um mindestens ein teilweise kondensiertes Kohlenwasserstoffgas bereitzustellen, das zu einem Separator (22-D1000) geschickt wird, um dadurch mindestens einen ersten Rückstanddampf (22) und mindestens eine C2 oder C3 enthaltende Flüssigkeit bereitzustellen, welche Flüssigkeit (25) auch leichtere Kohlenwasserstoffe enthält; und
(b) mindestens eine der C2 oder C3 enthaltenden Flüssigkeiten in eine Fraktioniersäule für Schwerfraktionen (22-T2100) geführt wird (25 bis 27 bis 28), worin die Flüssigkeit in einen zweiten Rückstand (34) getrennt wird, der leichtere Kohlenwasserstoffe und ein C2 oder C3 enthaltendes Produkt, enthält; umfassend
(1) Kühlen (22-E3200) des zweiten Rückstands (34), um den zweiten Rückstand teilweise zu kondensieren;
(2) inniges Kontaktieren mindestens eines Teils eines der ersten Rückstanddämpfe (22) mit mindestens einem Teil eines flüssigen Anteils des teilweise kondensierten zweiten Rückstands (30) in mindestens einer Kontaktierstufe (#1 #5 #10) einer Fraktioniersäule für Leichtfraktionen (22-T2000) und daraufhin Trennen von Dämpfen (39) und Flüssigkeiten (31) aus der Kontaktierstufe;
(3) Leiten der Dämpfe (39) aus der Kontaktierstufe (#1 #5 #10) der Fraktioniersäule für Leichtfraktionen (22-T2000) in ein Wärmetauschverhältnis (22-E3200) mit dem zweiten Rückstand (34) aus der Fraktioniersäule für Schwerfraktionen (22-T2100), um dadurch die Kühlung von Schritt (1) zu liefern und daraufhin Ablassen (39 bis 40) der Dämpfe aus der Kontaktierstufe der Fraktioniersäule für Leichtfraktionen (22-T2000);
(4) Abziehen eines Teils des ersten Rückstanddampfs (22);
(5) Kühlen (22-E3200) des Anteils des ersten Rückstanddampfs (22, 35), um den Anteil des ersten Rückstanddampfs teilweise zu kondensieren;
(6) inniges Kontaktieren (#1 #5 #10) mindestens eines Teils des ersten Dampfrückstands aus Schritt (2) mit mindestens einem Teil eines flüssigen Anteils des teilweise kondensierten Anteils des ersten Rückstands aus Schritt (5) in mindestens einer Kontaktierstufe der Fraktioniersäule für Leichtfraktionen (22-T2000) und daraufhin Trennen der Dämpfe (39) und Flüssigkeiten (31) aus der Kontaktierstufe;
(7) Abziehen eines Anteils der C2 oder C3 enthaltenden Flüssigkeit (25) aus dem Separator (22-D1000);
(8) Leiten (25, 26, 35) des Anteils der C2 oder C3 enthaltenden Flüssigkeit aus dem Separator (22-D1000) in ein Wärmetauschverhältnis (22-E3200) mit den Flüssigkeiten (31, 32) aus der Kontaktierstufe der Fraktioniersäule für Leichtfraktionen (22-T2000);
(9) Kühlen (22-E3200) des Anteils der C2 oder C3 enthaltenden Flüssigkeit (35) aus dem Separator (22-D1000) ;
(10) inniges Kontaktieren mindestens Teils eines der ersten Rückstanddämpfe (22, 23, 29) aus Schritt (2) mit mindestens einem Teil der C2 oder C3 enthaltenden Flüssigkeit (36) aus dem Separator (22-D1000) in der mindestens einen Kontaktierstufe (#1 #5 #10) der Fraktioniersäule für Leichtfraktionen (22-T2000) durch Zuführen (36, 37) des mindestens Teils eines der ersten Rückstanddämpfe mit mindestens einem Teil der C2 oder C3 enthaltenden Flüssigkeit zu der Fraktioniersäule für Leichtfraktionen (22-T2000) an einer Stelle, die höher (37) als (34, 38, 30) die Zufuhr des flüssigen Anteils des teilweise kondensierten zweiten Rückstands (38, 30) aus der Fraktioniersäule für Schwerfraktionen (22-T2100) liegt, und daraufhin Trennen der Dämpfe und Flüssigkeiten von der Kontaktierstufe;
(11) Liefern der Flüssigkeiten (32, 33) aus der Kontaktierstufe (#1 #5 #10) der Fraktioniersäule für Leichtfraktionen (22-T2000) aus Schritt (8) zu der Fraktioniersäule für Schwerfraktionen (22-T2100) als flüssige Zufuhr dazu
(12) Leiten der Dämpfe (39) aus der Kontaktierstufe (#1 #5 #10) der Fraktioniersäule für Leichtfraktionen (22-T2000) in ein Wärmetauschverhältnis (22-E3200) mit dem Anteil der ersten Rückstanddämpfe (22, 35) aus dem Separator (22-D1000), um dadurch das Kühlen von Schritt (5) zu liefern und daraufhin Ablassen (39, 40) der Dämpfe aus der Kontaktierstufe (#1 #5 #10) der Fraktioniersäule für Leichtfraktionen (22-T2000); und
(13) Leiten der Flüssigkeiten (31, 32) aus der Kontaktierstufe (#1 #5 #10) der Fraktioniersäule für Leichtfraktionen (22-T2000) in ein Wärmetauschverhältnis mit dem Anteil der C2 oder C3 enthaltenden Flüssigkeit (25, 26, 35) aus dem Separator (22-D1000), um dadurch die Kühlung von Schritt (9) zu liefern und daraufhin Ablassen der Flüssigkeiten (33) aus der Kontaktierstufe der Fraktioniersäule für Leichtfraktionen (22-T2000) zu der Fraktioniersäule für Schwerfraktionen (22-T2100).

2. Verfahren nach Anspruch 1, wobei der Kontaktierschritt (2) in einer Fraktioniersäule für Leichtfraktionen (22-T2000), die ein Fraktioniermittel für den Dampf/Flüssigkeits-Gegenstromkontakt enthält, ausgeführt wird und
(i) wobei der teilweise kondensierte zweite Rückstand (34) in die Fraktioniersäule für Leichtfraktionen (22-T2000) über oder an einer Zwischenstelle (30) in dem Fraktioniermittel der Fraktioniersäule für Leichtfraktionen (22-T2000) eingeführt wird, wodurch der flüssige Anteil davon abwärts durch das Fraktioniermittel hindurchgeht; und
(ii) wobei der teilweise kondensierte Anteil des ersten Rückstands (22, 35) in die Fraktioniersäule für Leichtfraktionen (22-T2000) über oder an einer Zwischenstelle (37) in dem Fraktioniermittel eingeführt wird, wobei der flüssige Anteil davon abwärts durch das Fraktioniermittel hindurchgeht; und wobei der Anteil der gekühlten C2 oder C3 enthaltenden Flüssigkeit (25) aus dem Separator (22-D1000) in die Fraktioniersäule für Leichtfraktionen (22-T2000) über oder an einer Zwischenstelle (37) in dem Fraktioniermittel eingeführt wird, wobei der flüssige Anteil davon abwärts durch das Fraktioniermittel hindurchgeht; und
(iii) der mindestens Teil eines der ersten Rückstanddämpfe (22, 35) zu der Fraktioniersäule für Leichtfraktionen (22-T2000) unterhalb des Fraktioniermittels geliefert wird, wobei der erste Rückstanddampf durch das Fraktioniermittel in Gegenstromkontakt mit dem flüssigen Anteil des teilweise kondensierten zweiten Rückstands aufsteigt.

3. Verfahren nach Anspruch 2, wobei das Fraktioniermittel in der Fraktioniersäule für Leichtfraktionen (22-T2000) das Äquivalent mindestens einer theoretischen Destillationsstufe bereitstellt, die zum Kontaktieren mindestens eines Teils eines der ersten Rückstanddämpfe (22, 23, 29) mit dem flüssigen Anteil des teilweise kondensierten zweiten Rückstands (34, 38, 30) angeordnet ist.

4. Verfahren nach Anspruch 1, wobei mindestens ein Teil eines der ersten Rückstanddämpfe (22, 23, 29) mit dem flüssigen Anteil des teilweise kondensierten zweiten Rückstands (34, 38, 30), dem flüssigen Anteil des teilweise kondensierten Anteils des ersten Rückstands und dem Anteil der gekühlten C2 oder C3 enthaltende Flüssigkeit aus dem Separator (22-D1000) zusammengemischt wird.

5. Verfahren nach Anspruch 1, wobei mindestens ein Teil eines der ersten Rückstanddämpfe in der Fraktioniersäule für Leichtfraktionen (22-T2000) mit sowohl den flüssigen Anteilen als auch den Dampfanteilen des teilweise kondensierten zweiten Rückstands, dem teilweise kondensierten Anteil des ersten Rückstanddampfs und dem Anteil der gekühlten C2 oder C3 enthaltende Flüssigkeit aus dem Separator (22-D1000) zusammengemischt wird.

6. System zur Trennung eines Kohlenwasserstoffgases enthaltend mindestens Methan, Ethan und C3-Komponenten in eine Fraktion, die einen vorwiegenden Anteil vom Methan oder Ethan und leichterer Komponenten enthält, und eine Fraktion, die einen vorwiegenden Anteil des C2 oder C3 und schwererer Komponenten enthält, dem Verfahren von Anspruch 1 entsprechend, in welchem System
(a) ein oder mehr erste Wärmetauschermittel (22-E3600, 22-E3700, 22-E3800) in Kommunikation mit einem oder mehr Ausdehnungsmittel (22-X6000) stehen, um mindestens ein teilweise kondensiertes Kohlenwasserstoffgas bereitzustellen, das zu einem Separator (22-D1000) geschickt wird, um dadurch mindestens einen ersten Rückstanddampf (22) und mindestens eine C2 oder C3 enthaltende Flüssigkeit (25) bereitzustellen, welche Flüssigkeit (25) auch leichtere Kohlenwasserstoffe enthält; und
(b) eine Fraktioniersäule für Schwerfraktionen (22-T2100), die zum Aufnehmen mindestens einer der C2 oder C3 enthaltenden Flüssigkeiten (33) angeschlossen ist, die geeignet ist, die C2 oder C3 enthaltenden Flüssigkeiten in einen zweiten Rückstand zu trennen, der leichtere Kohlenwasserstoffe und ein C2 oder C3 enthaltendes Produkt (34) enthält, ferner umfassend:
(1) zweite Wärmetauschermittel (22-E3200), die an die Fraktioniersäule für Schwerfraktionen (22-T2100) angeschlossen sind, um den zweiten Rückstand aufzunehmen und den zweiten Rückstand (34) teilweise zu kondensieren;
(2) wobei die zweiten Wärmetauschermittel (22-E3200) an den Separator (22-D1000) angeschlossen sind, um einen Anteil des ersten Rückstanddampfs (22) aufzunehmen und den Anteil des ersten Rückstands teilweise zu kondensieren;
(3) Fraktioniersäulenmittel für Leichtfraktionen (22-T2000), die angeschlossen sind, um mindestens einen Teil eines der ersten Rückstandsdämpfe (36) und mindestens einen Teil flüssiger Anteile aus dem teilweise kondensierten zweiten Rückstand (34) und teilweise kondensierten ersten Rückstanddampf (36) aufzunehmen und den mindestens einen Teil eines der ersten Rückstanddämpfe und die flüssigen Anteile des teilweise kondensierten zweiten Rückstands und teilweise kondensierten ersten Rückstanddampfs in mindestens einer Kontaktierstufe zusammenzumischen, wobei das Fraktioniersäulenmittel für Leichtfraktionen (22-T2000) Trennmittel (#1, #5, #10) zum Trennen des Dampfs und der Flüssigkeit aus der Kontaktierstufe nach Kontakt in der Stufe umfasst;
(4) das Fraktioniersäulenmittel für Leichtfraktionen (22-T2000) ferner angeschlossen ist, die Flüssigkeit aus der Kontaktierstufe zu der Fraktioniersäule für Schwerfraktionen (22-T2100) als flüssige Zufuhr dazu zu liefern;
(5) das Fraktioniersäulenmittel für Leichtfraktionen (22-T2000) auch angeschlossen ist, die Dämpfe aus der Kontaktierstufe (39) in ein Wärmetauschverhältnis mit dem zweiten Rückstand (36) und einem Anteil des ersten Rückstands (34) aus der Fraktioniersäule für Schwerfraktionen (22-T2100) bzw. dem Separator (22-D1000) in dem zweiten Wärmetauschermittel (22-E3200) zu führen; und
(6) drittes Wärmetauschermittel (22-D1100) an die Fraktioniersäule für Schwerfraktionen (22-T2100) angeschlossen ist, um die Flüssigkeiten aus der Kontaktierstufe (33) aufzunehmen und den Anteil der C2 oder C3 enthaltende Flüssigkeit aus dem Separator (22-D1000) zu kühlen.

7. Vorrichtung nach Anspruch 6, wobei das Fraktioniersäulenmittel für Leichtfraktionen (22-T2000) Fraktioniermittel (#1, #5, #10) für den Gegenstromdampf/Flüssigkeitskontakt umfasst und wobei das Mittel zum Aufnehmen des Anteils eines der ersten Rückstanddämpfe angeschlossen ist, die darin unter dem Fraktioniermittel behandelt werden sollen, und zum Aufnehmen des Anteils der Flüssigkeiten aus dem teilweise kondensierten zweiten Rückstand, des Anteils des teilweise kondensierten ersten Rückstands und Anteils der gekühlten C2 oder C3 enthaltenden Flüssigkeit aus dem Separator (22-D1000), die drin über oder an einer Zwischenstelle in dem Fraktioniermittel behandelt werden soll, wobei das Fraktioniermittel dadurch so eingestellt wird, dass die ersten Rückstanddämpfe dort hindurch in Gegenstromkontakt mit teilweise kondensiertem zweitem Rückstand und einem Anteil des teilweise kondensierten ersten Rückstands aufsteigen, und ferner eingestellt wird, so dass der Anteil der C2 oder C3 enthaltenden Flüssigkeit aus dem Separator durch die Flüssigkeiten, die aus dem Fraktioniermittel austreten, gekühlt wird.

8. Vorrichtung nach Anspruch 7, wobei die Fraktioniermittel (#1, #5, #10) Dampf/Flüssigkeitskontaktiermittel umfassen, die mindestens einer theoretischen Destillationsstufe äquivalent sind.

9. Vorrichtung nach Anspruch 6, wobei das Fraktioniersäulenmittel für Leichtfraktionen (22-T2000) Mittel zum Zusammenmischen mindestens eines Teils eines der ersten Rückstanddämpfe mit dem flüssigen Anteil des teilweise kondensierten zweiten Rückstands, des flüssigen Anteils des teilweise kondensierten Anteils des ersten Rückstands und dem Anteil der gekühlten C2 oder C3 enthaltende Flüssigkeit aus dem Separator (22-D1000) umfasst.

10. Vorrichtung nach Anspruch 6, wobei das Fraktioniersäulenmittel für Leichtfraktionen (22-T2000) Mittel zum Zusammenmischen mindestens eines Teils eines der ersten Rückstanddämpfe mit sowohl dem flüssigen als auch dem Dampfanteil des teilweise kondensierten zweiten Rückstands, des teilweise kondensierten Anteils des ersten Rückstands und dem Anteil der gekühlten C2 oder C3 enthaltende Flüssigkeit aus dem Separator (22-D1000) umfasst.

## Revendications

1. Procédé pour la séparation d'un gaz d'hydrocarbures contenant au moins du méthane, de l'éthane et des composants en C3 en une fraction contenant une majeure partie de l'éthane et de composants plus légers et une fraction contenant une majeure partie des composants en C3 et plus lourds ou une majeure partie du méthane et de composants plus légers et une fraction contenant une majeure partie des composants en C2 et plus lourds, dans lequel procédé
(a) le gaz d'alimentation (20) est traité dans un ou plusieurs échangeurs de chaleur (22-E3600, 22-E3700, 22-E3800) et des étapes de détente pour fournir au moins un gaz d'hydrocarbures partiellement condensé, qui est envoyé vers un séparateur (22-D1000) ce qui fournit de cette manière au moins une vapeur de premier résidu (22) et au moins un liquide contenant des C2 ou des C3 lequel liquide (25) contient également des hydrocarbures plus légers ; et
(b) au moins l'un des liquides contenant des C2 ou des C3 est acheminé (25 vers 27 vers 28) vers une colonne de fractionnement de fractions de queue lourdes (22-T2100) dans laquelle ledit liquide est séparé en un second résidu (34) contenant des hydrocarbures plus légers et un produit contenant des C2 ou des C3 ; comprenant :
(1) le refroidissement (22-E3200) dudit second résidu (34) pour condenser partiellement ledit second résidu ;
(2) la mise en contact intime d'au moins une partie de l'une desdites vapeurs de premier résidu (22) avec au moins une partie d'une partie liquide du second résidu partiellement condensé (30) dans au moins un étage de mise en contact (#1 #5 #10) d'une colonne de fractionnement de fractions de queue légères (22-T2000) et par la suite la séparation de vapeurs (39) et de liquides (31) provenant dudit étage de mise en contact ;
(3) l'acheminement des vapeurs (39) provenant dudit étage de mise en contact (#1 #5 #10) de la colonne de fractionnement de fractions de queue légères (22-T2000) vers une relation d'échange de chaleur (22-E3200) avec ledit second résidu (34) provenant de la colonne de fractionnement de fractions de queue lourdes (22-T2100), pour de cette manière assurer le refroidissement de l'étape (1), et par la suite l'évacuation (39 vers 40) desdites vapeurs provenant dudit étage de mise en contact de la colonne de fractionnement de fractions de queue légères (22-T2000) ;
(4) le soutirage d'une partie de la vapeur de premier résidu (22) ;
(5) le refroidissement (22-E3200) de ladite partie de la vapeur de premier résidu (22, 35) pour condenser partiellement ladite partie de ladite vapeur de premier résidu ;
(6) la mise en contact intime (#1 #5 #10) de l'au moins une partie de l'une desdites vapeurs de premier résidu provenant de l'étape (2) avec au moins une partie d'une partie liquide de la partie partiellement condensée du premier résidu provenant de l'étape (5) dans l'au moins un étage de mise en contact de la colonne de fractionnement de fractions de queue légères (22-T2000) et par la suite la séparation des vapeurs (39) et des liquides (31) provenant dudit étage de mise en contact ;
(7) le soutirage d'une partie du liquide contenant des C2 ou des C3 (25) du séparateur (22-D1000) ;
(8) l'acheminement (25, 26, 35) de ladite partie du liquide contenant des C2 ou des C3 provenant du séparateur (22-D1000) vers une relation d'échange de chaleur (22-E3200) avec les liquides (31, 32) provenant de l'étage de mise en contact de la colonne de fractionnement de fractions de queue légères (22-T2000) ;
(9) le refroidissement (22-E3200) de ladite partie du liquide contenant des C2 ou des C3 (35) provenant du séparateur (22-D1000) ;
(10) la mise en contact intime de l'au moins une partie de l'une desdites vapeurs de premier résidu (22, 23, 29) de l'étape (2) avec au moins une partie du liquide contenant des C2 ou des C3 (36) provenant du séparateur (22-D1000) dans l'au moins un étage de mise en contact (#1, #5 #10) de la colonne de fractionnement de fractions de queue légères (22-T2000) par l'alimentation (36, 37) de la colonne de fractionnement de fractions de queue légères (22-T2000) en l'au moins une partie de l'une desdites vapeurs de premier résidu avec au moins une partie du liquide contenant des C2 ou des C3 en un point plus élevé (37) que l'alimentation (34, 38, 30) en la partie liquide du second résidu partiellement condensé (38, 30) provenant de la colonne de fractionnement de fractions de queue lourdes (22-T2100) et par la suite la séparation des vapeurs et des liquides provenant dudit étage de mise en contact ;
(11) l'apport des liquides (32, 33) provenant dudit étage de mise en contact (#1 #5 #10) de la colonne de fractionnement de fractions de queue légères (22-T2000) de l'étape (8) à la colonne de fractionnement de fractions de queue lourdes (22-T2100) en tant que charge d'alimentation liquide de celle-ci ;
(12) l'acheminement des vapeurs (39) provenant dudit étage de mise en contact (#1 #5 #10) de la colonne de fractionnement de fractions de queue légères (22-T2000) vers une relation d'échange de chaleur (22-E3200) avec ladite partie des vapeurs de premier résidu (22, 35) provenant du séparateur (22-D1000), pour de cette manière assurer le refroidissement de l'étape (5), et par la suite l'évacuation (39, 40) desdites vapeurs provenant dudit étage de mise en contact (#1 #5 #10) de la colonne de fractionnement de fractions de queue légères (22-T2000) ; et
(13) l'acheminement des liquides (31, 32) provenant dudit étage de mise en contact (#1 #5 #10) de la colonne de fractionnement de fractions de queue légères (22-T2000) vers une relation d'échange de chaleur avec ladite partie du liquide contenant des C2 ou des C3 (25, 26, 35) provenant du séparateur (22-D1000), pour de cette manière assurer le refroidissement de l'étape (9), et par la suite l'évacuation desdits liquides (33) provenant dudit étage de mise en contact de la colonne de fractionnement de fractions de queue légères (22-T2000) vers la colonne de fractionnement de fractions de queue lourdes (22-T2100).

2. Procédé selon la revendication 1 dans lequel ladite étape de mise en contact (2) est effectuée dans une colonne de fractionnement de fractions de queue légères (22-T2000) qui comprend un moyen de fractionnement pour le contact vapeur/liquide à contre-courant et
(i) dans lequel ledit second résidu partiellement condensé (34) est introduit dans ladite colonne de fractionnement de fractions de queue légères (22-T2000) au-dessus ou en un point intermédiaire (30) dans ledit moyen de fractionnement de la colonne de fractionnement de fractions de queue légères (22-T2000), moyennant quoi la partie liquide de celui-ci passe en descendant dans ledit moyen de fractionnement ; et
(ii) dans lequel ladite partie partiellement condensée du premier résidu (22, 35) est introduite dans ladite colonne de fractionnement de fractions de queue légères (22-T2000) au-dessus ou en un point intermédiaire (37) dans ledit moyen de fractionnement, moyennant quoi la partie liquide de celle-ci passe en descendant dans ledit moyen de fractionnement ; et dans lequel ladite partie du liquide contenant des C2 ou des C3 refroidi (25) provenant du séparateur (22-D1000) est introduite dans ladite colonne de fractionnement de fractions de queue légères (22-T2000) au-dessus ou en un point intermédiaire (37) dans ledit moyen de fractionnement, moyennant quoi la partie liquide de celle-ci passe en descendant dans ledit moyen de fractionnement ; et
(iii) ladite au moins une partie de l'une desdites vapeurs de premier résidu (22, 35) est apportée à ladite colonne de fractionnement de fractions de queue légères (22-T2000) au-dessous dudit moyen de fractionnement, moyennant quoi la vapeur de premier résidu monte dans ledit moyen de fractionnement en contact à contre-courant avec la partie liquide du second résidu partiellement condensé.

3. Procédé selon la revendication 2 dans lequel le moyen de fractionnement dans ladite colonne de fractionnement de fractions de queue légères (22-T2000) fournit l'équivalent d'au moins un étage de distillation théorique conçu pour mettre en contact au moins une partie de l'une desdites vapeurs de premier résidu (22, 23, 29) avec la partie liquide du second résidu partiellement condensé (34, 38, 30).

4. Procédé selon la revendication 1 dans lequel au moins une partie de l'une desdites vapeurs de premier résidu (22, 23, 29) est co-mélangée avec la partie liquide du second résidu partiellement condensé (34, 38, 30), une partie liquide de la partie partiellement condensée du premier résidu et une partie du liquide contenant des C2 ou des C3 refroidi provenant du séparateur (22-D1000).

5. Procédé selon la revendication 1 dans lequel au moins une partie de l'une desdites vapeurs de premier résidu est co-mélangée dans la colonne de fractionnement de fractions de queue légères (22-T2000) avec à la fois les parties liquides et les parties de vapeur dudit second résidu partiellement condensé, une partie partiellement condensée de la vapeur de premier résidu et une partie du liquide contenant des C2 ou des C3 refroidi provenant du séparateur (22-D1000).

6. Système pour la séparation d'un gaz d'hydrocarbures contenant au moins du méthane, de l'éthane et des composants en C3 en une fraction contenant une majeure partie de méthane ou d'éthane et de composants plus légers et une fraction contenant une majeure partie des composants en C2 ou C3 et plus lourds selon le procédé de la revendication 1 dans lequel système
(a) un ou plusieurs premiers moyens d'échange de chaleur (22-E3600, 22-E3700, 22-E3800) sont en communication avec un ou plusieurs moyens de détente (22-X6000) pour fournir au moins un gaz d'hydrocarbures partiellement condensé qui est envoyé vers un séparateur (22-D1000), ce qui fournit de cette manière au moins une vapeur de premier résidu (22) et au moins un liquide contenant des C2 ou des C3 (25) lequel liquide contient également des hydrocarbures plus légers ; et
(b) une colonne de fractionnement de fractions de queue lourdes (22-T2100) raccordée pour recevoir au moins l'un desdits liquides contenant des C2 ou des C3 (33) qui est conçue pour séparer les liquides contenant des C2 ou des C3 en un second résidu contenant des hydrocarbures plus légers et un produit contenant des C2 ou des C3 (34) ; comprenant en outre :
(1) un deuxième moyen d'échange de chaleur (22-E3200) raccordé à ladite colonne de fractionnement de fractions de queue lourdes (22-T2100) pour recevoir ledit second résidu et pour condenser partiellement ledit second résidu (34) ;
(2) ledit deuxième moyen d'échange de chaleur (22-E3200) raccordé audit séparateur (22-D1000) pour recevoir ladite partie de la vapeur de premier résidu (22) et pour condenser partiellement ladite partie dudit premier résidu ;
(3) un moyen colonne de fractionnement de fractions de queue légères (22-T2000) raccordé pour recevoir au moins une partie de l'une des vapeurs de premier résidu (36) et au moins une partie de parties liquides provenant du second résidu partiellement condensé (34) et de vapeur de premier résidu partiellement condensée (36) et pour co-mélanger ladite au moins une partie de l'une des vapeurs de premier résidu et les parties liquides du second résidu partiellement condensé et de vapeur de premier résidu partiellement condensée dans au moins un étage de mise en contact, ledit moyen colonne de fractionnement de fractions de queue légères (22-T2000) comprenant un moyen de séparation (#1, #5, #10) pour la séparation de la vapeur et du liquide provenant dudit étage de mise en contact après le contact dans ledit étage ;
(4) ledit moyen colonne de fractionnement de fractions de queue légères (22-T2000) étant en outre raccordé pour apporter le liquide provenant dudit étage de mise en contact à la colonne de fractionnement de fractions de queue lourdes (22-T2100) en tant que charge d'alimentation liquide de celle-ci ;
(5) ledit moyen colonne de fractionnement de fractions de queue légères (22-T2000) étant également raccordé pour acheminer les vapeurs provenant dudit étage de mise en contact (39) vers une relation d'échange de chaleur avec ledit second résidu (36) et une partie du premier résidu (34) provenant de la colonne de fractionnement de fractions de queue lourdes (22-T2100) et du séparateur (22-D1000) respectivement dans ledit deuxième moyen d'échange de chaleur (22-E3200) ; et
(6) un troisième moyen d'échange de chaleur (22-D1100) raccordé à ladite colonne de fractionnement de fractions de queue lourdes (22-T2100) pour recevoir lesdits liquides provenant dudit étage de mise en contact (33) et pour refroidir la partie du liquide contenant des C2 ou des C3 provenant du séparateur (22-D1000).

7. Appareil selon la revendication 6 dans lequel ledit moyen colonne de fractionnement de fractions de queue légères (22-T2000) comprend un moyen de fractionnement (#1, #5, #10) pour le contact vapeur/liquide à contre-courant et dans lequel ledit moyen est raccordé pour recevoir la partie de l'une des vapeurs de premier résidu à traiter dans celui-ci au-dessous dudit moyen de fractionnement et pour recevoir la partie desdits liquides provenant du second résidu partiellement condensé, une partie du premier résidu partiellement condensé et une partie du liquide contenant des C2 ou des C3 refroidi provenant du séparateur (22-D1000) à traiter dans celui-ci au-dessus ou en un point intermédiaire dans ledit moyen de fractionnement, ledit moyen de fractionnement étant de cette manière adapté pour que les vapeurs de premier résidu montent dans celui-ci en contact à contre-courant avec du second résidu partiellement condensé et une partie du premier résidu partiellement condensé et étant en outre adapté pour que la partie du liquide contenant des C2 ou des C3 provenant du séparateur soit refroidie par les liquides sortant du moyen de fractionnement.

8. Appareil selon la revendication 7 dans lequel ledit moyen de fractionnement (#1, #5, #10) comprend un moyen de mise en contact vapeur/liquide qui est l'équivalent d'au moins un étage de distillation théorique.

9. Appareil selon la revendication 6 dans lequel ledit moyen colonne de fractionnement de fractions de queue légères (22-T2000) comprend un moyen pour le co-mélange d'au moins une partie de l'une desdites vapeurs de premier résidu avec la partie liquide du second résidu partiellement condensé, une partie liquide de la partie partiellement condensée du premier résidu et une partie du liquide contenant des C2 ou des C3 refroidi provenant du séparateur (22-D1000).

10. Appareil selon la revendication 6 dans lequel ledit moyen colonne de fractionnement de fractions de queue légères (22-T2000) comprend un moyen pour le co-mélange d'au moins une partie de l'une desdites vapeurs de premier résidu avec à la fois la partie liquide et la partie vapeur dudit second résidu partiellement condensé, ladite partie partiellement condensée du premier résidu et une partie du liquide contenant des C2 ou des C3 refroidi provenant du séparateur (22-D1000).
